# EUROPEAN PATENT SPECIFICATION

(11) **EP 3 865 046 B1**
(45) Date of publication and mention of the grant of the patent: **03.09.2025**
(21) Application number: 20305131.3
(22) Date of filing: 12.02.2020
(51) Int. Cl.: A61B 3/00

(54) **DETECTING AND CORRECTING A VARIATION OF CURRENT REFRACTIVE ERROR AND OF CURRENT ACCOMMODATION AMPLITUDE OF A PERSON**
DETEKTION UND KORREKTUR EINER VARIATION EINES AKTUELLEN REFRAKTIVEN FEHLERS UND DER AKTUELLEN AKKOMMODATIONSAMPLITUDE EINER PERSON
DÉTECTION ET CORRECTION D'UNE VARIATION D'ERREUR DE RÉFRACTION ET D'AMPLITUDE D'ACCOMMODATION ACTUELLES D'UNE PERSONNE

(43) Date of publication of application: 18.08.2021
(73) Proprietor: ESSILOR INTERNATIONAL, 94220 Charenton-Le-Pont (FR)
(72) Inventor: MARIN, Gildas, 94220 CHARENTON-LE-PONT (FR); NETTER, Estelle, 94220 CHARENTON-LE-PONT (FR); GIRAUDET, Guillaume, 94220 CHARENTON-LE-PONT (FR)
(74) Representative: Plasseraud IP

(56) References cited:
- US-A9- 2019 142 267

## Description

### FIELD OF THE INVENTION

The present invention relates in general to optical articles, and in particular, to methods, computer programs, computer storage media, processing circuits and optical equipments for detecting a variation of a current refractive error or a variation of a current accommodation amplitude of a person, and to methods, computer programs, computer storage media, processing circuits and optical equipments for correcting a refractive error or a lack of accommodation amplitude of a person.

### BACKGROUND OF THE INVENTION

Between two appointments at an eye care practitioner (ECP), one's refractive error may naturally evolve.

Common refractive errors include myopia, hyperopia and astigmatism.

Myopia corresponds to the eye focusing light before, instead of on, the retina. As a result, far objects appear blurry.

Hyperopia corresponds to the eye focusing light behind, instead of on, the retina. As a result, close objects appear blurry.

Astigmatism corresponds to the eye not focusing light evenly on the retina. As a result, both close objects and far objects appear blurry.

It has been shown in particular that when the myopia of a person becomes undercorrected, meaning the current prescription value for compensating said myopia by means of an optical equipment has progressed in comparison to an initial prescription value corresponding to that of the optical equipment usually worn by the person, their myopia progresses quicker than if it was perfectly corrected.

Myopia is a common refractive error, currently affecting about one third of the population. High myopia is associated with higher risk of retinal and choroidal pathologies. Several methods and products are used to slow down myopia progression. Among these solutions, orthokeratology contact lenses, soft bifocal contact lenses, topical pharmaceutical agents such as atropine or pirenzepine, and progressive or bifocal ophthalmic lenses have been shown to be more or less effective, through randomized controlled trials.

Slowing down myopia progression as soon as it appears is crucial, and may potentially benefit millions of children and adults worldwide.

A solution for detecting myopia progression as soon as it appears is regularly scheduling appointments at an ECP. However, this solution is not very practical. Even during controlled trials, visits for refractive error assessment take place every 6 to 12 months, at best. Several months after each ECP exam and the new eyeglasses, because of myopia progression, myopic children are often exposed to blurred visual environment. Their current eyeglasses are not properly adapted to their new refraction anymore. Outside these clinical studies, in daily life, 18 to 24 months can separate two eye exams. Long periods between visits may be due to eye care professional unavailability or inaccessibility and also to blur tolerance in children. Children may be exposed to substantial defocus before notifying their parents. Numerous studies showed that the more children are exposed to blurred scenes, the higher the speed of myopia progression.

Another common eye condition, assimilated in this document to a type of refractive error, is presbyopia. Presbyopia is a normal part of the aging process, thus anybody is eventually affected.

Due to aging of the eye, the eye tends to focus light behind, instead of on, the retina. As a result of insufficient accommodation amplitude, the ability to focus clearly on close objects progressively worsens. As a result, close objects appear blurry.

Detecting presbyopia progression and possibly adapting a provided correction as presbyopia progresses may potentially benefit millions of adults worldwide.

It is proposed in WO2018213010 to adjust the power of a variable lens, via the measurement of the refractive defect of the user, via an autorefractometer included in the device. Nevertheless, this solution does not provide the wanted effect on refraction evolution detection because an autorefractometer is not see-through, thus cannot be continuously worn to continuously provide measurements. Moreover using an objective measurement for measurement refraction is known to be inaccurate, so the refraction provided by the variable lens may not perfectly correct user visual aberration. This can lead to an increase of refraction defect instead of a reduction of refraction evolution.

It is further disclosed in US2019/142267 a known method for estimating future axial elongation of an individual's eye. In this context, there is a need to estimate the evolution of the refractive error of a person at an appropriate time. Depending on this evolution, there is also a need to determine an updated power correction suitable for correcting the current refractive error of the person.

### SUMMARY OF THE INVENTION

Embodiments of the invention provide a method for detecting a variation of a current refractive error of a person, the method comprising:
a) obtaining an optical equipment comprising an optical lens, a dioptric function of the optical lens being adapted for correcting an initial refractive error of a person, the optical equipment further comprising a processing circuit comprising a processor operably connected to a memory,
b) using the memory, obtaining a current value of a parameter, the current value being indicative of a variation of a current refractive error of the person with respect to the initial refractive error, and
c) using the processing circuit, detecting a variation of a current refractive error of the person with respect to the initial refractive error based on the obtained current value of the parameter.

By "refractive error" are included any of myopia, hyperopia, astigmatism, presbyopia and combinations thereof. In the case of presbyopia, the "refractive error" actually indicates a lack of accommodation amplitude.

By initial refractive error is understood a refractive error at a reference point in time.

By current refractive error is understood a refractive error at a current point in time, the current point in time being later than the reference point in time.

Embodiments of the invention further provide a processing circuit comprising a processor operably connected to a memory, the processing circuit being configured to implement the above method.

Embodiments of the invention further provide an optical equipment comprising an optical lens, a dioptric function of the optical lens being adapted for correcting an initial refractive error of a person, and the above processing circuit.

The optical equipment may further comprise one or more sensors adapted to obtain said current value of a parameter, and the processing circuit may further comprise a communication interface with said one or more sensors.

Embodiments of the invention further provide a computer program comprising one or more stored sequence/s of instructions that is accessible to a processor and which, when executed by the processor, causes the processor to carry out the steps of one of the above methods.

Embodiments of the invention further provide a storage medium, or "memory", storing one or more stored sequence/s of instructions of the above computer program.

The methods, computer programs, storage media, processing circuits, sensors and optical equipments above allow detecting a variation of a current refractive error with respect to an initial refractive error of a person. This effect is achieved in particular due to the common feature of obtaining, using a memory, a current value of a parameter, the current value being indicative of said variation.

It is thus possible, to determine, for example by a processing circuit integrated in the eyewear usually worn by the person, whether a dioptric function of an optical lens of the eyewear is still adapted or not to the current refractive error of the person. If not, it is possible to determine an updated dioptric function suitable to compensate for the current refractive error of the person.

For example, as a result, an evolution of myopia for a child may be monitored and, the dioptric function suitable to compensate said myopia may be updated, without the need to schedule frequent visits to clinicians and while simplifying the correct assessment of the refraction of the eye over known methods as the assessment is performed without needing any input from the child.

In an embodiment, the processor and the memory are operably connected to a first sensor which, when the optical equipment is worn by the person, is adapted to measure the value of said parameter, and the method further comprises:
- using the first sensor, measuring the current value of said parameter, the optical equipment being worn by the person.

The first sensor allows evaluating in situ the evolution of the refractive error of the person. Therefore the optical equipment is a standalone device able to evaluate said evolution.

In embodiments, the first sensor is adapted to measure a physiological parameter of a person wearing the optical equipment, the physiological parameter being indicative of the variation of the current refractive error of the person with respect to the initial refractive error.

Measuring or monitoring physiological parameters of the person wearing the optical equipment provides objective values which may be gathered and analyzed to estimate the evolution of the refractive error of the person.

In an embodiment:
- the first sensor is an eye squinting sensor,
- the physiological parameter is an occurrence of eye squinting of the person wearing the optical equipment, and
- measuring the current value of said parameter comprises detecting, using the eye squinting sensor, an occurrence of eye squinting of the person wearing the optical equipment.

An eye squinting sensor is non-invasive and allows recording indications that a current vision of the person wearing the optical equipment may have become blurred. Recording such indication may help detecting, for example, that the vision of a child has degraded even without them actively identifying this degradation.

In an embodiment:
- the first sensor comprises at least one electrode adapted for electroencephalography,
- each of the at least one electrode is arranged to be in contact with a frontal or an occipital area of the person when the optical equipment is worn by the person,
- the physiological parameter is an electrical activity of the brain of the person wearing the optical equipment, and
- measuring the current value of said parameter comprises analyzing, using the at least one electrode, the electrical activity of the brain of the person wearing the optical equipment.

Electrodes for electroencephalography are non-invasive and are already present on some commercially available head-mounted accessories for various purposes. Recording and analyzing brain waves over time allow detecting neuronal activity and associating a higher prevalence of neuronal activity, or a higher number of occurrences of neuronal activity in some viewing situations, to an increased accommodation or perceived blur by the person wearing the optical equipment. Said increased accommodation or perceived blur is an indication that the current refractive error of the person has evolved with respect to an initial refractive error.

In an embodiment:
- the memory stores at least one indication of a reference viewing condition,
- the optical equipment further comprises at least one second sensor which, when the optical equipment is worn by the person, is adapted to measure said viewing condition parameter,
- the method further comprises obtaining, by the at least one second sensor, at least one indication of a current viewing condition, and
- detecting a variation of a current refractive error of the person with respect to the initial refractive error is further based on a comparison between the at least one indication of the current viewing condition and the at least one indication of the reference viewing condition.

The reference viewing condition allows putting the measurement from the first sensor into perspective by comparing measurements only when they correspond to similar viewing situations as detected by the second sensor.

In an embodiment, at least one second sensor is a light condition sensor and the viewing condition parameter is indicative of a light intensity of a scene viewed by the person through the active refractive lens while measuring, by the first sensor, a current value of the parameter.

Measuring the light intensity of the scene allows detecting a bright atmosphere, in which case, for example, eye squinting may be non-related to an evolution of refractive error.

In an embodiment, at least one second sensor is a distance sensor and the viewing condition parameter is indicative of a distance between the active refractive lens and an object viewed by the person through the active refractive lens while measuring, by the first sensor, a current value of the parameter.

By doing so, it is possible to determine, at a current instant, whether the person wearing the optical equipment is undergoing a far, intermediate or near vision activity. Therefore, any measurement by the first sensor at the current instant may be associated to a far vision activity, to an intermediate vision activity or to a near vision activity. Thus, it is possible to determine the evolution over time of, for example, eye squinting, or brain waves, specifically during near, intermediate, or far vision activities. An evolution of a specific eye defect may thus accurately be monitored by selecting only the measurement from the first sensor during vision activities corresponding to a specific vision distance.

In an embodiment, the optical lens is an active refractive lens and the method further comprises:
using the processing circuit, generating an alert to the person if the detected variation exceeds a predetermined threshold.

The alert may prompt the person to control the active refractive lens so as to adjust the correction to fit their needs.

Embodiments of the invention further provide a method for adjusting a dioptric function of an active refractive lens comprising:
a) obtaining an optical equipment comprising an active refractive lens, a dioptric function of the active refractive lens being adapted for correcting an initial refractive error of a person, the optical equipment further comprising a processing circuit comprising a processor operably connected to a memory and to a communication interface with the active refractive lens,
b) using the memory, obtaining a current value of a parameter, the current value being indicative of a variation of a current refractive error of the person with respect to the initial refractive error,
c) using the processing circuit, detecting a variation of a current refractive error of the person with respect to the initial refractive error based on the obtained current value of the parameter, and
d) using the processing circuit, adjusting the dioptric function of the active refractive lens if the detected variation exceeds a predetermined threshold, and maintaining the dioptric function of the active refractive lens if the detected variation is below, or equal to, the predetermined threshold.

Embodiments of the invention further provide a processing circuit comprising a processor operably connected to a memory and to a communication interface with an active refractive lens, the processing circuit being configured to implement the above method for correcting a refractive error of a person.

Embodiments of the invention further provide an optical equipment comprising an active refractive lens and the above processing circuit. The optical equipment may further comprise one or more sensors adapted to obtain said current value of a parameter, and the processing circuit may further comprise a communication interface with said one or more sensors.

Embodiments of the invention further provide a computer program comprising one or more stored sequence/s of instructions that is accessible to a processor and which, when executed by the processor, causes the processor to carry out the steps of the above method for correcting a refractive error of a person.

Embodiments of the invention further provide a storage medium, or "memory", storing one or more stored sequence/s of instructions of the above computer program.

The methods above for correcting a refractive error of a person, the computer programs, storage media, processing circuits and optical equipments therefor all allow automatically driving an active refractive lens for correcting a refractive error, or an accommodation amplitude, of a person while accounting for an evolution of said refractive error over time. Therefore, the person always gets the right correction even when their refractive error evolves, without needing to perform an eye examination at an eye care practitioner.

In an embodiment, the optical equipment comprises one or more sensors, the processing circuit comprises a communication interface with the one or more sensors, the communication interface being operably coupled with the processor and the memory, and obtaining the current value of the parameter comprises measuring said current value of the parameter using the one or more sensors.

In an embodiment, measuring or obtaining the current value of said parameter and detecting the variation of the current refractive error of the person with respect to the initial refractive error are repeated over time.

Therefore, the evolution of the current refractive error of the person is monitored over time, which allows detecting an evolution of the current refractive error with respect to the initial refractive error, as soon as possible after said evolution occurs. It is thus possible to alert that the refractive error of the person has evolved, or to directly correct their current refractive error, accounting for said evolution, soon after said evolution occurs.

In an embodiment, adjusting the dioptric function of the active refractive lens comprises adjusting one or more of a sphere component, a cylinder component, an axis component, an addition and a prism component.

Adjustment of the sphere component allows correcting nearsightedness / farsightedness. Adjustments of the cylinder component and/or axis allow correcting astigmatism. Adjustment of the addition allows correcting presbyopia.

Adjustment of the prism allows compensating for eye alignment issues, which may not have been detected in the past or may have been induced by the dioptric change of the active refractive lenses.

In an embodiment, the method comprises:
- using the memory, obtaining a predictive model of a variation of refractive error of the person over time, and
- using the processing circuit, predicting the current value of the parameter based on the predictive model.

For example, the optical equipment may comprise a sensor, the parameter may be repeatedly measured by the sensor over time to form a database and the predictive model may be established based on the database so as to predict future measurements.

Predicting future measurements allows double checking future measurements in order to detect and indicate, for example, a malfunction of a sensor.

Alternately, the optical equipment may be devoid of sensors for measuring said refractive error over time and the predictive model stored on the memory may be based on sociological data. For example, a predictive model may be obtained based on a recorded evolution of refractive error over time for a cohort of persons, in which case the predictive model is also collaborative. Such predictive model allows anticipating an evolution of a refractive error and always provide an up-to-date correction even without needing to measure said refractive error over time.

### BRIEF DESCRIPTION OF THE DRAWINGS

For a more complete understanding of the description provided herein and the advantages thereof, reference is now made to the brief descriptions below, taken in connection with the accompanying drawings and detailed description, wherein like reference numerals represent like parts.
FIG. 1 depicts an example of a processing circuit adapted for implementing a method according to an embodiment of the invention.
FIG. 2 depicts an example of a general flowchart of a method according to an embodiment of the invention, for detecting a variation of current refractive error of a person,
FIG. 3 depicts an example of a general flowchart of a method according to an embodiment of the invention, for correcting a variation of current refractive error of a person,
FIG. 4 depicts an example of a general flowchart of a method according to an embodiment of the invention, for detecting and correcting a variation of current refractive error of a person, and
FIG. 5 and FIG. 6 depict examples of optical equipments according to embodiments of the invention.

### DETAILED DESCRIPTION OF THE INVENTION

In the description which follows the drawing figures are not necessarily to scale and certain features may be shown in generalized or schematic form in the interest of clarity and conciseness or for informational purposes. In addition, although making and using various embodiments are discussed in detail below, it should be appreciated that as described herein are provided many inventive concepts that may be embodied in a wide variety of contexts. Embodiments discussed herein are merely representative and do not limit the scope of the invention. It will also be obvious to one skilled in the art that all the technical features that are defined relative to a process can be transposed, individually or in combination, to a system and conversely, all the technical features relative to a system can be transposed, individually or in combination, to a process.

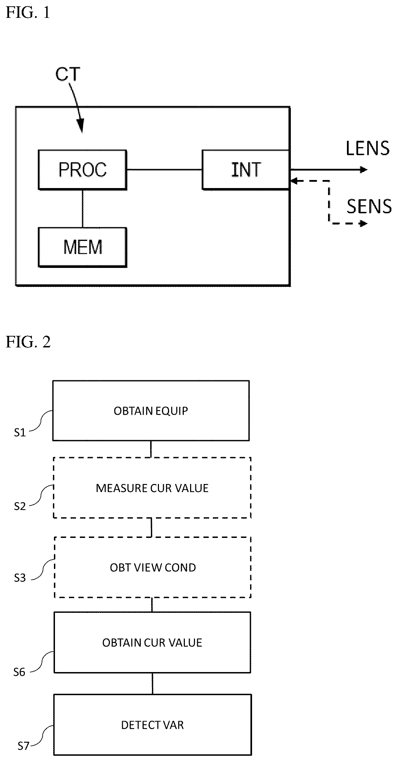

It is now referred to [FIG. 1], which depicts an example of a processing circuit according to embodiments of the invention, the processing circuit being adapted to implement a method for detecting and/or for correcting a variation of current refractive error of a person.

The processing circuit comprises a processor (PROC) operably coupled to a memory (MEM).

In an embodiment of the invention, the processing circuit further comprises a communication interface (INT) operably coupled with the processor (PROC).

The communication interface (INT) may be configured to communicate with one or more sensors (SENS).

The communication interface (INT) may be configured to communicate with one or more active refractive lenses (LENS).

The communication interface may be wired and/or wireless. Different types of wired or wireless technologies are applicable and their respective advantages are well known from the skilled person.

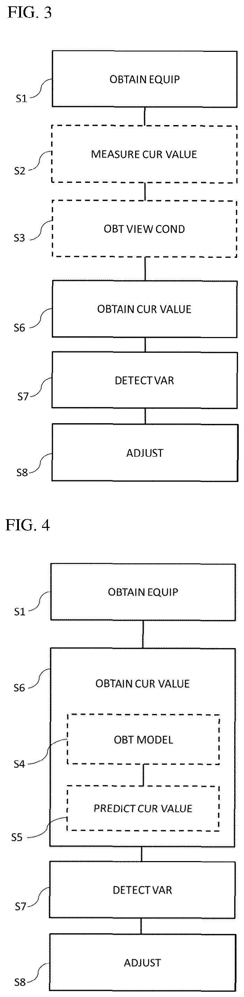

It is now referred to [FIG. 2], [FIG. 3] and [FIG. 4] which each depict an example of a general flowchart of a method according to exemplary embodiments of the invention, for respectively:
- detecting a variation of current refractive error of a person ([FIG. 2]) based on sensing a current value of a parameter indicating said variation,
- detecting said variation, as in the embodiment of [FIG. 2], and correcting said variation by adjusting an active dioptric function of an active refractive lens ([FIG. 3]), and
- detecting said variation by using a predictive model and correcting said variation by adjusting an active dioptric function of an active refractive lens ([FIG. 4]).

According to each of the examples as illustrated in [FIG. 2], [FIG. 3] and [FIG. 4], an optical equipment is obtained OBTAIN EQUIP (S1).

The optical equipment comprises an optical lens with a dioptric function adapted for correcting an initial refractive error of an eye of a person.

Of course, the optical equipment may comprise a pair of optical lenses, the dioptric function of the optical lenses being adapted for correcting an initial refractive error of both eyes of the person.

The optical equipment further comprises the processing circuit described above and depicted in [FIG. 1].

The memory (MEM) stores a computer program to be executed by the processor (PROC). The general flowchart of the computer program comprises at least, as illustrated in [FIG. 2], [FIG. 3] and [FIG. 4]:
- obtaining a current value OBTAIN CUR VALUE (S6) of a parameter, stored on the memory (MEM), the current value being indicative of a variation of a current refractive error of the person with respect to the initial refractive error, and
- based on the obtained current value of the parameter, detecting, by the processing circuit (CT), a variation DETECT VAR (S7) of said current refractive error of the person with respect to said initial refractive error of the person.

The variation of the current refractive error of the given person with respect to the initial refractive error may relate to a monocular refractive error or to a binocular refractive error.

Various possibilities to obtain said current value of the parameter (S6) may be implemented and are described hereafter.

For example, said current value of the parameter may be directly measured, by a first sensor (SENS) incorporated in the obtained optical equipment, and stored on the memory (MEM).

For example, said current value of the parameter may be indirectly determined by the processing circuit (CT) from a measurement by at least one first sensor (SENS) incorporated in the optical equipment, the measurement being stored on the memory (MEM).

For example, said current value of the parameter may be indirectly determined by the processing circuit (CT) from a combination of measurements by at least one first sensor (SENS) and by at least one second sensor both incorporated in the optical equipment, both measurements being stored on the memory (MEM). For example, a combined measurement of gaze distance, surrounding luminous flux and palpebral openness allows predicting the evolution of refractive error of the person.

For example, said current value of the parameter may be determined by the processing circuit (CT) from an obtained predictive model.

It is now referred to exemplary embodiments as illustrated in [FIG. 2], in which:
- the obtained optical equipment comprises one or more first sensors adapted to measure said current value of the parameter,
- the obtained optical equipment may further comprise one or more second sensors adapted to measure a current viewing condition,
- the current value of the parameter is obtained OBTAIN CUR VALUE (S6) based on inputs from at least one or more first sensors incorporated in the obtained optical equipment, and
- the variation of the current refractive error of the person with respect to the initial refractive error is detected DETECT VAR (S7) based at least on the obtained current value of the parameter.

The at least one first sensor (SENS) is instructed by the processing circuit (CT) to measure MEASURE CUR VALUE (S2) a current value of a parameter. The thusly obtained current value is transmitted to the processing circuit (CT) and stored on the memory (MEM).

In embodiments, measuring the current value of the parameter MEASURE CUR VALUE (S2) relates to directly measuring a variation of a current refractive error of a given person with respect to their initial refractive error.

Said current value of a parameter may be recorded for example by a first sensor comprising a combination of one or more infrared (IR) light sources such as lightemitting diodes (LED) emitting at 860 nm, placed in the branches of the optical equipment and of IR sensors placed on the optical equipment such as cameras being sensitive in the IR light range and being focused so that a sharp image of the eye of the given person may be acquired. Each IR sensor is close to a corresponding IR light source, so as to measure IR light that has been emitted by the IR light source and reflected on the observer's sclera.

The optical equipment may further comprise a reflective element, for instance an holographic mirror on the back surface of the refractive lens, designed to reflect toward the eye the light coming from the IR light source. Preferably, the reflective element is such that the light rays coming to the eye are collimated, so that they enter the eye pupil being parallel, but this configuration is not mandatory and the light can be diverging or converging (between +-3D for instance).

According to the general principle of eccentric photorefraction, the emitted light enters the eye, reaches the retina, and is then reflected by the retina toward the reflective element, then reflected back to the camera, so that an image of the eye of the given person is obtained. On the obtained image, a light crescent is seen on the pupil of the given person. The size of that light crescent depends on the refractive error of the given person.

In embodiments, measuring the current value of the parameter MEASURE CUR VALUE (S2) relates to detecting a behavioral and/or a physiological change that can be induced by blurred vision.

In embodiments, the detected behavioral and physiological blur-induced change is an indication of a brain wave variation.

Indeed, it is well known that decision making mainly takes place in the frontal part of the brain. As decision making becomes more and more difficult with increasing level of blur, in far vision activities, the pattern of brain activity changes.

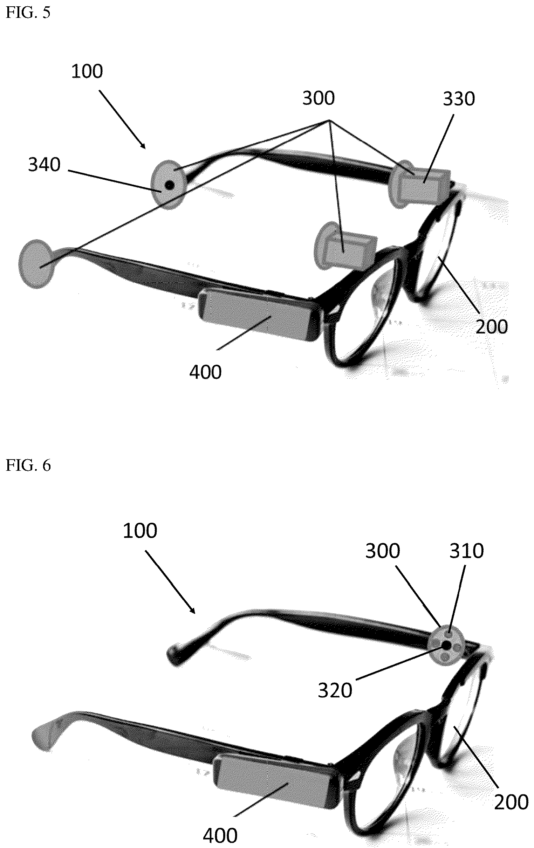

It is now referred to [FIG. 5] which depicts an example of an obtained optical equipment adapted to record a pattern of brain activity of a person over time.

The obtained optical equipment 100 comprises a pair of optical lenses 200 mounted on a spectacle frame and further comprises a first sensor 300. The first sensor 300 comprises:
- two dry electroencephalography (EEG) sensors 330 placed at the top of the frame of the optical equipment, in contact with skull regions close to frontal cortical areas when the optical equipment 100 is worn by the person, and, optionally,
- two dry EEG sensors 340 placed at the extremity of eyeglasses branches in order to record brain activity in primary visual areas when the optical equipment 100 is worn by the person.

The obtained optical equipment 100 further comprises one or more second sensors 400 for measuring an additional parameter related to the wearer and/or to the current vision activity.

These sensors may record cortical activity, continuously or during several minutes at different periods of the day and for various visual tasks. The recorded cortical activity may be analyzed, for example using an algorithm following deeplearning principles, to provide a feedback, in real time or at the end of the day, about the level of defocus (or "blur") experienced by the wearer. It may be optionally considered, based on the recorded cortical activity over time, to generate a behavioral baseline, representative of the usual visual habits of the wearer under an optimal visual correction, and to use the generated behavioral baseline as an indication of whether a current measurement of cortical activity indicates a current occurrence of an experienced defocus or not. For instance, brain activity signals recorded during the days and weeks following the last eye exam and the wear of the new equipment, may be used as baseline.

For near vision, the cortical signal of blurred vision may be used as biofeedback to force the person, for example a child, to accommodate exactly on the reading plane. For far vision activities, the cortical signal of blurred vision may indicate as feedback that myopia has progressed since the last eye exam and that new eyeglasses should be provided to the person. This feedback may be given at the end of each day, every couple of days, every week, etc.

In embodiments, the detected behavioral and physiological blur-induced change is an indication of eyes squinting propensity.

Eyes squinting enables to voluntary decrease the pupil size. This behavior is used, for example, to protect against bright glaring light - by reducing the amount of light entering the eyes. It is also well known that eyes squinting enables getting improved visual acuity when an observer is exposed to optical defocus. Indeed, narrowing the palpebral fissure decreases the pupil diameter and improves the depth of focus. The increase in prevalence of eyes squinting during far vision tasks is a good marker of myopia progression. When myopia progresses, images are not perfectly focused on the retina anymore. Hence, a person whose myopia has progressed to be undercorrected more often squints its eyes and decreases the level of blur to be able to read at far distance. Using big-data statistics and/or deep learning principles, increase in squinting propensity can be detected in visual situations that should not require that kind of behavior, that is in far vision and normal lighting conditions for a myopic person, or in near vision and normal lighting conditions for a hyperopic or presbyopic person.

It is now referred to [FIG. 6] which depicts an example of an obtained optical equipment adapted to record a change in eyes squinting propensity throughout time.

The obtained optical equipment 100 comprises a pair of optical lenses 200 mounted on a spectacle frame and further comprises a first sensor 300. The first sensor 300 comprises a combination of one or more IR LED 310 placed in the branches of the optical equipment and of one or more IR sensors 320 placed on the optical equipment so as to measure IR light that has been emitted by the IR LED and reflected on the observer's sclera. A global decrease in the reflected IR light indicates an increase in squinting propensity.

Alternately, electromyography (EMG) sensors may be placed on a portion of the eyewear equipment that is in contact with the person's temples, close to the eyes. The EMG sensors may thus record an increase in palpebral muscles strength, which indicates an increase in squinting propensity.

Hence, when it is detected that eyes squinting propensity of the person increases for far vision activities and in normal light conditions, it can be concluded that the current visual correction of the person is no longer adapted, that myopia has increased and that an updated visual correction is needed.

Measuring the current value of the parameter MEASURE CUR VALUE (S2) using any of the first sensors as described above in connection with [FIG. 2] and [FIG. 3] results in obtaining OBTAIN CUR VALUE (S6) said current value of the parameter, which is then stored on the memory (MEM).

Said stored current value of the parameter may be directly interpreted for detecting DETECT VAR (S7) the variation of current refractive error of a person.

Better interpretation of the current value of the parameter can, however, be obtained if said current value is reliably associated to a current wearer condition (such as a head posture, and/or an eye gaze direction, and/or a viewing distance, etc.) and/or to a current environment condition (such as a light condition, and/or a time of day, etc.). Associating recordings to current wearer or environment conditions allows comparing any obtained recording with a previous obtained recording under similar wearer or environment conditions. Thus, any detected deviation may be interpreted as being linked to the variation of the user eye refraction only.

Many different sensors may be used, the examples provided and detailed above are non-limitative. It should also be noted, that even if the examples above are related to determining an evolution of myopia, each can also apply to other types of visual impairments, such as presbyopia, hypermetropia, astigmatism, etc.

In case of myopia, people may squint during a far vision activity because of difficulty to see clear at a far distance but not during a near vision activity.

In case of presbyopia or hypermetropia, people may squint during a near vision activity if they have difficulties to accommodate but not during a far vision activity. Age may be an additional information allowing to distinguish between hyperopia and presbyopia. For example, a person squinting during near vision activities may be considered hyperopic if aged less than for example 40 or 45, and presbyopic instead.

In case of astigmatism, people squint because of difficulties to see clear whatever the viewing distance is.

In embodiments, the obtained optical equipment comprises at least one second sensor adapted to obtain a current viewing condition. The at least one second sensor (SENS) is instructed by the processing circuit (CT) to obtain OBT VIEW COND (S3) a current viewing condition. The thusly obtained current viewing condition is transmitted to the processing circuit (CT), associated to the stored current value of the parameter, and stored on the memory (MEM).

The viewing condition may be related to the wearer (wearer-related viewing condition or "wearer condition") or to the environment of the wearer (environmentrelated viewing condition or "environment condition").

For example, a sensor, such as a telemeter, may be used to inform about the viewing distance so that the processing circuit may determine whether the wearer is undergoing a far or a near vision activity. Such viewing distance sensor may for instance be used in combination to brain activity recording sensors, in order to best analyze the evolution of brain activity over time and in order to identify, for example, whether the person experiences blur or not in far distance viewing activities.

Moreover, knowledge of whether the given person is undergoing a far or a near vision activity allows determining whether a recorded change in the refraction of the eye of the given person is induced, for example, by myopia progression or by accommodation. Thanks to this telemeter device, combined with a continuous recording of changes in the refraction of the eyes of the given person, it is possible to obtain statistics of accommodation response and inform about potential accommodation lag in near vision. Numerous studies have shown that the higher the accommodation lag, the faster the myopia progression. These statistics of accommodation response might be used to precociously detect a risk of myopia progression, even before myopia actually progresses.

Beyond helping determining the progression of the refractive error of a wearer, such sensor might also be useful to check whether the refractive error is well corrected in various and long lasting daily activities.

For example, a camera directed to an eye of the wearer may detect the eyegaze direction of the wearer. The obtained eyegaze direction may be associated by the processing circuit to an inferred gazing distance using an ergorama. A lowered gazing can for example be associated to near vision. A straight away gazing may for example be associated to far vision.

For example, a light intensity sensor may be used to provide to the processing circuit an indication of a light level in the environment of the wearer.

For example an accelerometer may be combined with a distance sensor to indicate, at a given instant, that the eyewear is tilted and the distance is short. The processing circuit may determine, based on these combined indications, that the wearer is undergoing a reading activity. On the contrary, when the eyewear is currently not tilted and the gazing distance corresponds to far vision, the processing circuit may determine that the wearer is undergoing a far vision related activity.

Based on the stored current viewing condition, it is possible, among multiple and/or continuous recordings of values of a parameter, to specifically select recordings corresponding to similar wearer and environment conditions.

Such a sensor is particularly relevant in combination with an eyes squinting sensor (since eye squinting may depend on light level).

The current viewing condition obtained OBT VIEW COND (S3) using any of the second sensors as described above in connection with [FIG. 2] and [FIG. 3], and stored on the memory (MEM) may be interpreted in combination with the stored current value of the parameter for detecting DETECT VAR (S7) the variation of current refractive error of a person.

Various possibilities to detect DETECT VAR (S7) the variation of said current refractive error of the person with respect to said initial refractive error of the person may be implemented.

For example, the obtained current value of the parameter may be compared to a predetermined threshold. Such comparison may help monitoring a continuous evolution of refractive error of the given person. For example, such comparison may indicate whether the current refractive error of the given person remains or not within a tolerance band around the initial refractive error.

For example, the current value of the parameter may be repeatedly obtained over time. The variation over time of the current value of the parameter may be compared to a predetermined threshold. Such comparison may for example indicate a sudden, unexpected, evolution of refractive error of the given person.

For example, an evolution of a refraction of the eye of the person may be determined based on multiple/continuous recordings selected among a larger number of different recordings so that they show similar wearer and environment conditions. Such determination allows comparing only recordings corresponding to situations representative of the evolution of a specific refractive error, which allows overcoming the accuracy difficulty to get precise refraction evolution and also allows saving memory and processing resources by only considering relevant measurements from any first sensor incorporated in the obtained optical equipment. For example, in the case of myopia, only measurements from first sensors during far vision activities need to be considered. For example, in the case of presbyopia, only measurements from first sensors during far vision activities need to be considered.

In embodiments, after detecting DETECT VAR (S7) a variation of the current refractive error of the person with respect to the initial refractive error, the processing circuit (CT) generates an alert, to the person or to a third party.

The alert may be used to signal said detected variation and, for example, order a new optical equipment or schedule an appointment with an eye care practitioner.

In embodiments, based on detecting DETECT VAR (S7) a variation of a current refractive error of the person with respect to the initial refractive error, the processing circuit (CT) issues a signal for adjusting the correction power of at least one optical lens of the optical equipment to compensate for said detected variation.

The signal can be an alert signal destined to provide for example a visual or audio alert to the person wearing the optical equipment, so that the person manually adjusts said correction power.

Alternately the signal can be a command signal for automatically adjusting said correction power.

It is now referred to [FIG. 3], in which:
- a variation of a current refractive error of the person with respect to the initial refractive error is detected DETECT VAR (S7) according to any of the embodiments described above in connection with [FIG. 2],
- at least one optical lens of the obtained optical equipment is an active refractive lens, and
- the detected variation of the current refractive error of the person with respect to the initial refractive error is used to adjust ADJUST (S8) the correction power provided by the active refractive lens to compensate for said detected variation.

Of course, detecting DETECT VAR (S7) and adjusting ADJUST (S8) can be repeated throughout time in order to repeatedly / continuously providing an up-to-date correction power to the person.

In an embodiment of the invention, the obtained optical equipment is a pair of smart eyeglasses, comprising:
- a pair of active refractive lenses,
- at least one first sensor adapted to measure current values of a parameter indicating a variation of a current refractive error of a given person with respect to their initial refractive error,
- at least one second sensor adapted to measure a current viewing condition, and
- a computer system implementing a method as illustrated in [FIG. 3].

An active refractive lens is an optical lens exhibiting a total lens correction power which may be described as the combination of a first lens correction power adapted to central vision correction and of a second lens correction power, which is active (in other words, can be controlled) and which provides to the given person an additional central vision correction or a peripheral correction.

The first lens correction power can be either passive or active and is normally initially adapted to the initial refraction, for example the first lens correction power is equal to a prescription value prescribed by an eye care practitioner for the given person.

The active correction power can be obtained with different technologies.

In large field Alvarez lenses, two plates are able to slide across one another. Depending on the relative position of both plates, a different correction power is obtained. Driving can be manual thanks to different commands (eg: sliders, rollers, ...). In this case, the time when the correction power must be changed and the amount of correction power to add may be communicated to the given person via any kind of human machine interface (HMI) (directly on the optical equipment, via an application, via a warning on a terminal such as a smartphone, ...). Driving can be automatic via electrically-driven motors. In this case, the needed correction power may be directly modified and the wearer be informed.

In large field fluidic lenses, a shape of a cavity filled with a fluid is controlled to provide a variable correction power.

For example, the optical equipment may comprise a pump to control the amount of liquid in a cavity possibly encapsulated between two ophthalmic shells and made of an elastic membrane on one side. When fluid is injected inside the cavity the elastic membrane is deformed, which changes the power of the lens.

For example, the lens may comprise a cavity containing a fluid possibly encapsulated between two ophthalmic shells. The cavity is made of an elastic membrane. The optical equipment may further comprise mechanical actuators distributed along a deformable ring that hold the membrane under tension. The mechanical actuators will deform the deformable ring in several points that will deform the membrane and change the power of the lens.

For example, the lens may comprise an assembly of a first cavity filled with a first liquid having a first refractive index, a second cavity filled with a second liquid having a second refractive index different from the first refractive index, and an elastic membrane as separation between the first cavity and the second cavity. The assembly may be encapsulated in ophthalmic shells having a refractive index which may for example be close to the first refractive index. The optical equipment may further comprise a reversible pump for regulating the relative amounts of the first liquid and the second liquid in order to deform the elastic membrane.

In any kind of large field fluidic lenses, driving can be manual thanks to different commands (eg: sliders, rollers, ...). In this case, the time when the correction power must be changed and the amount of correction power to add may be communicated to the given person via any kind of human machine interface (HMI) (directly on the optical equipment, via an application, via a warning on a terminal such as a smartphone, ...). Driving can be automatic via electrically-driven actuators or pumps. In this case, the needed correction power may be directly modified and the given person be informed.

Other technologies of active refractive lenses are possible, such as liquid crystal electro-optic lenses comprising liquid crystals arranged according to a pixelated matrix. Each liquid crystal may be controlled in order to provide a specific power correction. Liquid crystal electro-optic lenses allow providing a finely controlled optical design. Driving may be automatic via electrically-driven active/passive matrices. In this case, the needed correction power may be directly modified and the wearer be informed.

Based on an initial refraction of the eyes of a given person, the dioptric function of each active refractive lens is initially set so as to correct the initial refractive error, if any, of the given person. The initial refraction may be obtained by running any standard or known refraction determination process.

Adjusting ADJUST (S8) the correction power provided by the active refractive lens to compensate for the detected variation allows always providing the adequate correction to a person whose refraction error evolves throughout time, as detected based on the measurements over time by the first sensors and, optionally, by the second sensors.

Thus, the optical equipment provides a correction which is adapted to the given person in that it is accounted for the evolution of the refractive error of the given person with respect to the initial refractive error.

Various modifications of the concept as described above may be admitted. In particular, it is not necessary for the obtained optical equipment to comprise any sensors. It is also not necessary to collect any measurements over time in order to assess the evolution of the refractive error of the person and to provide a correction accounting to this evolution.

It is now referred to [FIG. 4], in which the obtained optical equipment comprises at least an active refractive lens and a processing circuit (CT). This implementation may be cost-effective and weight-effective as the optical equipment may be devoid of any sensors.

A predictive model of a variation of refractive error of the person over time OBT MODEL (S4) is obtained by the processing circuit (CT). For a myopia predictive model, input parameters can be chosen among but not restricted to: age, ethnicity, gender, previous ECP measured prescription value (including sphere, cylinder or mean sphere(s)). For a presbyopia predictive model, input parameters can be chosen among but not restricted to: age, sun exposure habits, previous ECP measured prescription values (including sphere, cylinder or mean sphere(s), addition).

In embodiments, the current value of a parameter indicative of a variation of a current refractive error of the given person with respect to their initial refractive error PREDICT CUR VALUE (S5) is predicted by the processing circuit (CT).

Predicting said current value may be based on the obtained predictive model.

Said current value of the parameter, indicating a variation of a current refractive error of the person with respect to the initial refractive error is thus obtained OBTAIN CUR VALUE (S6) based on the stored predictive model rather than on measurements,

The variation of the current refractive error of the person with respect to the initial refractive error is detected DETECT VAR (S7) based on the obtained current value of the parameter

The detected variation of the current refractive error of the person with respect to the initial refractive error is used to adjust ADJUST (S8) the correction power provided by the active refractive lens to compensate for said predicted variation.

Thus, the optical equipment provides a correction which is adapted to the given person in that it is accounted for the evolution of the refractive error of the given person with respect to the initial refractive error.

The embodiments described above in connection with [FIG. 2] and [FIG. 4] respectively may be combined with each other.

In particular, the predictive model described above in connection with [FIG. 4] may be combined with the measurements from the first sensors, and optionally from the second sensors as described above in connection with [FIG. 2].

Indeed, the predictive model can be improved and personalized to the given person using collected data obtained from repeatedly performing direct measurements of refractive error evolution of the given person over time. When the measurement of refractive error evolution is obtained indirectly by combining the obtained measurements from the first sensor and the second sensor, the quality of the prediction from the predictive model can also be improved using collected data obtained from repeatedly performing measurements using the first sensor and the second sensor over time and combining said obtained measurements.

## Claims

1. Method for detecting a variation of a current refractive error of a person, the method comprising:
a) obtaining (S1) an optical equipment adapted to be worn by the person and comprising an optical lens, a dioptric function of the optical lens being adapted for correcting an initial refractive error of the person, the optical equipment further comprising a processing circuit comprising a processor operably connected to a memory,
b) using the memory, obtaining (S6) a current value of a parameter, the current value being indicative of a variation of a current refractive error of the person with respect to the initial refractive error,
c) using the processing circuit, detecting (S7) a variation of a current refractive error of the person with respect to the initial refractive error based on the obtained current value of the parameter, and
d) using the processing circuit, generating an alert when the detected variation exceeds a predetermined threshold.

2. Method according to the preceding claim, the method further comprising:
- using the memory, obtaining (S4) a predictive model of a variation of refractive error of the
person over time, and
- using the processing circuit, predicting (S5) the current value of the parameter based on the predictive model.

3. Method according to any of the preceding claims, wherein the processor and the memory are operably connected to a first sensor which, when the optical equipment is worn by the person, is adapted to measure the value of said parameter, and the method further comprises:
- using the first sensor, measuring (S2) the current value of said parameter, the optical equipment being worn by the person.

4. Method according to claim 3, wherein the first sensor is a physiological sensor adapted to measure a physiological parameter of a person wearing the optical equipment, the physiological parameter being indicative of the variation of the current refractive error of the person with respect to the initial refractive error.

5. Method according to claim 4, wherein:
- the physiological sensor is an eye squinting sensor,
- the physiological parameter is an occurrence of eye squinting of the person wearing the optical equipment, and
- measuring (S2) the current value of said parameter comprises detecting, using the eye squinting sensor, an occurrence of eye squinting of the person wearing the optical equipment.

6. Method according to claim 4, wherein:
- the physiological sensor comprises at least one electrode adapted for electroencephalography,
- each of the at least one electrode is arranged to be in contact with a frontal or an occipital area of the person when the optical equipment is worn by the person,
- the physiological parameter is an electrical activity of the brain of the person wearing the optical equipment, and
- measuring (S2) the current value of said parameter comprises analyzing, using the at least one electrode, the electrical activity of the brain of the person wearing the optical equipment.

7. Method according to any of claims 3 to 6, wherein:
- the memory stores an indication of a reference viewing condition,
- the optical equipment further comprises at least one second sensor which, when the optical equipment is worn by the person and the person is viewing at a scene through the optical equipment, is adapted to obtain an indication of a current viewing condition,
- the method further comprises, the optical equipment being worn by the person and the person being viewing at the scene through the optical equipment, obtaining (S3), by the second sensor, the indication of the current viewing condition, and
- detecting (S7) a variation of a current refractive error of the person with respect to the initial refractive error is further based on a comparison between the indication of the current viewing condition and the indication of the reference viewing condition.

8. Method according to claim 7, wherein at least one second sensor is a light condition sensor and the viewing condition parameter is indicative of a light intensity of the scene viewed by the person through the active refractive lens while measuring, by the physiological sensor, a current value of the physiological parameter.

9. Method according to claim 7, wherein at least one second sensor is a distance sensor and the viewing condition parameter is indicative of a distance between the active refractive lens and an object of the scene viewed by the person through the active refractive lens while measuring, by the physiological sensor, a current value of the physiological parameter.

10. Method according to any of claims 3 to 9, wherein measuring (S2) the current value of said parameter and detecting (S7) the variation of the current ametropia of the person with respect to the initial ametropia are repeated over time.

11. Method for adjusting a dioptric function of an active refractive lens, comprising:
a) obtaining (S1) a see-through optical equipment comprising an active refractive lens, a dioptric function of the active refractive lens being adapted for correcting an initial refractive error of a person, the optical equipment further comprising a processing circuit comprising a processor operably connected to a memory and to a communication interface with the active refractive lens,
b) using the memory, obtaining (S6) a current value of a parameter, the current value being indicative of a variation of a current refractive error of the person with respect to the initial refractive error,
c) using the processing circuit, detecting (S7) a variation of a current refractive error of the person with respect to the initial refractive error based on the obtained current value of the parameter, and
d) using the processing circuit, adjusting (S8) the dioptric function of the active refractive lens if the detected variation exceeds a predetermined threshold, and maintaining the dioptric function of the active refractive lens if the detected variation is below, or equal to, the predetermined threshold.

12. Optical equipment comprising an optical lens and a processing circuit, the processing circuit comprising a processor (PROC) connected to a memory (MEM), the processing circuit being configured to implement steps b) to d) of the method of any of claims 1 to 11,
wherein the optical equipment is see-through and adapted to be worn by a person.

## Patentansprüche

1. Verfahren zur Detektion einer Variation eines aktuellen Brechungsfehlers einer Person, wobei das Verfahren Folgendes umfasst:
a) Erhalten (S1) einer optischen Ausrüstung, die dafür ausgelegt ist, von der Person getragen zu werden, und eine optische Linse umfasst, wobei eine dioptrische Funktion der optischen Linse dafür ausgelegt ist, einen anfänglichen Brechungsfehler der Person zu korrigieren, wobei die optische Ausrüstung ferner eine Verarbeitungsschaltung umfasst, die einen wirksam mit einem Speicher verbundenen Prozessor umfasst,
b) Erhalten (S6) eines aktuellen Werts eines Parameters unter Verwendung des Speichers, wobei der aktuelle Wert eine Variation eines aktuellen Brechungsfehlers der Person in Bezug auf den anfänglichen Brechungsfehler angibt,
c) Detektieren (S7) einer Variation eines aktuellen Brechungsfehlers der Person in Bezug auf den anfänglichen Brechungsfehler unter Verwendung der Verarbeitungsschaltung auf der Basis des erhaltenen aktuellen Werts des Parameters und
d) Erzeugen eines Alarms unter Verwendung der Verarbeitungsschaltung, wenn die detektierte Variation eine vorbestimmte Schwelle überschreitet.

2. Verfahren nach dem vorhergehenden Anspruch, wobei das Verfahren ferner Folgendes umfasst:
- Erhalten (S4) eines prädiktiven Modells einer Variation eines Brechungsfehlers der Person im Verlauf der Zeit unter Verwendung des Speichers und
- Vorhersagen (S5) des aktuellen Werts des Parameters unter Verwendung der Verarbeitungsschaltung auf der Basis des Vorhersagemodells.

3. Verfahren nach einem der vorhergehenden Ansprüche, wobei der Prozessor und der Speicher wirksam mit einem ersten Sensor verbunden sind, der, wenn die optische Ausrüstung von der Person getragen wird, dafür ausgelegt ist, den Wert des Parameters zu messen, und das Verfahren ferner Folgendes umfasst:
- Messen (S2) des aktuellen Werts des Parameters unter Verwendung des ersten Sensors, wobei die optische Ausrüstung von der Person getragen wird.

4. Verfahren nach Anspruch 3, wobei der erste Sensor ein physiologischer Sensor ist, der dafür ausgelegt ist, einen physiologischen Parameter einer die optische Ausrüstung tragenden Person zu messen, wobei der physiologische Parameter die Variation des aktuellen Brechungsfehlers der Person in Bezug auf den anfänglichen Brechungsfehler angibt.

5. Verfahren nach Anspruch 4, wobei
- der physiologische Sensor ein Augenquetschsensor ist,
- der physiologische Parameter ein Auftreten von Augenquetschen der die optische Ausrüstung tragenden Person ist und
- Messen (S2) des aktuellen Werts dieses Parameters Detektion eines Auftretens eines Augenquetschens der die optische Ausrüstung tragenden Person unter Verwendung des Augenquetschsensors umfasst.

6. Verfahren nach Anspruch 4, wobei
- der physiologische Sensor mindestens eine Elektrode umfasst, die für Elektroenzephalographie ausgelegt ist,
- jede der mindestens einen Elektrode so angeordnet ist, dass sie mit einem frontalen oder einem occipitalen Bereich der Person in Kontakt steht, wenn die optische Ausrüstung von der Person getragen wird;
- der physiologische Parameter eine elektrische Aktivität des Gehirns der die optische Ausrüstung tragenden Person ist und
- Messen (S2) des aktuellen Werts des Parameters das Analysieren der elektrischen Aktivität des Gehirns der die optische Ausrüstung tragenden Person unter Verwendung der mindestens einen Elektrode umfasst.

7. Verfahren nach einem der Ansprüche 3 bis 6, wobei
- der Speicher eine Angabe einer Referenzbetrachtungsbedingung speichert,
- die optische Ausrüstung ferner mindestens einen zweiten Sensor umfasst, der, wenn die optische Ausrüstung von der Person getragen wird und die Person an einer Szene durch die optische Ausrüstung betrachtet, dafür ausgelegt ist, eine Angabe einer aktuellen Betrachtungsbedingung zu erhalten,
- das Verfahren ferner, während die optische Ausrüstung von der Person getragen wird und die Person, die durch die optische Ausrüstung an der Szene betrachtet, Erhalten (S3) der Angabe des aktuellen Betrachtungszustands durch den zweiten Sensor umfasst und
- Detektion (S7) einer Variation eines aktuellen Brechungsfehlers der Person in Bezug auf den anfänglichen Brechungsfehler ferner auf einem Vergleich zwischen der Angabe der aktuellen Betrachtungsbedingung und der Angabe der Referenzbetrachtungbedingung basiert.

8. Verfahren nach Anspruch 7, wobei mindestens ein zweiter Sensor ein Lichtbedingungssensor ist und der Betrachtungsbedingungsparameter eine Lichtintensität der Szene angibt, die von der Person durch die aktive refraktive Linse betrachtet wird, während durch den physiologischen Sensor ein aktueller Wert des physiologischen Parameters gemessen wird.

9. Verfahren nach Anspruch 7, wobei mindestens ein zweiter Sensor ein Abstandssensor ist und der Betrachtungsbedingungsparameter einen Abstand zwischen der aktiven refraktiven Linse und einem Objekt der Szene angibt, das von der Person durch die aktive refraktive Linse betrachtet wird, während durch den physiologischen Sensor ein aktueller Wert des physiologischen Parameters gemessen wird.

10. Verfahren nach einem der Ansprüche 3 bis 9, wobei Messen (S2) des aktuellen Werts des Parameters und Detektion (S7) der Variation der aktuellen Ametropie der Person in Bezug auf die anfängliche Ametropie im Verlauf der Zeit wiederholt werden.

11. Verfahren zum Anpassen einer dioptrischen Funktion einer aktiven refraktiven Linse, das Folgendes umfasst:
a) Erhalten (S1) einer optischen Durchsichtausrüstung, die eine aktive refraktive Linse umfasst, wobei eine dioptrische Funktion der aktiven refraktiven Linse zum Korrigieren eines anfänglichen refraktiven Fehlers einer Person ausgelegt ist, wobei die optische Ausrüstung ferner eine Verarbeitungsschaltung umfasst, die einen wirksam mit einem Speicher und mit einer Kommunikationsschnittstelle mit der aktiven refraktiven Linse verbundenen Prozessor umfasst,
b) Erhalten (S6) eines aktuellen Werts eines Parameters unter Verwendung des Speichers, wobei der aktuelle Wert eine Variation eines aktuellen Brechungsfehlers der Person in Bezug auf den anfänglichen Brechungsfehler angibt,
c) Detektieren (S7) einer Variation eines aktuellen Brechungsfehlers der Person in Bezug auf den anfänglichen Brechungsfehler unter Verwendung der Verarbeitungsschaltung auf der Basis des erhaltenen aktuellen Werts des Parameters und
d) Anpassen (S8) der dioptrischen Funktion der aktiven refraktiven Linse unter Verwendung der Verarbeitungsschaltung, wenn die detektierte Variation eine vorbestimmte Schwelle überschreitet, und Beibehalten der dioptrischen Funktion der aktiven refraktiven Linse, wenn die detektierte Variation kleiner oder gleich der vorbestimmten Schwelle ist.

12. Optische Ausrüstung, die eine optische Linse und eine Verarbeitungsschaltung umfasst, wobei die Verarbeitungsschaltung einen mit einem Speicher (MEM) verbundenen Prozessor (PROC) umfasst, wobei die Verarbeitungsschaltung dafür ausgelegt ist, die Schritte b) bis d) des Verfahrens nach einem der Ansprüche 1 bis 11 zu implementieren,
wobei die optische Ausrüstung durchsichtig ist und dafür ausgelegt ist, von einer Person getragen zu werden.

## Revendications

1. Procédé de détection d'une variation d'une erreur de réfraction courante chez une personne, le procédé comprenant :
a) l'obtention (S1) d'un équipement optique adapté à être porté par la personne et comprenant une lentille optique, une fonction dioptrique de la lentille optique étant adaptée à corriger une erreur de réfraction initiale chez la personne, l'équipement optique comprenant en outre un circuit de traitement comprenant un processeur relié fonctionnellement à une mémoire,
b) à l'aide de la mémoire, l'obtention (S6) d'une valeur courante d'un paramètre, la valeur courante indiquant une variation d'une erreur de réfraction courante chez la personne par rapport à l'erreur de réfraction initiale,
c) à l'aide du circuit de traitement, la détection (S7) d'une variation d'une erreur de réfraction courante chez la personne par rapport à l'erreur de réfraction initiale sur la base de la valeur courante obtenue du paramètre, et
d) à l'aide du circuit de traitement, la génération d'une alerte lorsque la variation détectée dépasse un seuil prédéterminé.

2. Procédé selon la revendication précédente, le procédé comprenant en outre :
- à l'aide de la mémoire, l'obtention (S4) d'un modèle prédictif d'une variation d'erreur de réfraction chez la personne dans le temps, et
- à l'aide du circuit de traitement, la prédiction (S5) de la valeur courante du paramètre sur la base du modèle prédictif.

3. Procédé selon l'une quelconque des revendications précédentes, dans lequel le processeur et la mémoire sont reliés fonctionnellement à un premier capteur qui, lorsque l'équipement optique est porté par la personne, est adapté à mesurer la valeur dudit paramètre, et le procédé comprenant en outre :
- à l'aide du premier capteur, la mesure (S2) de la valeur courante dudit paramètre, l'équipement optique étant porté par la personne.

4. Procédé selon la revendication 3, dans lequel le premier capteur est un capteur physiologique adapté à mesurer un paramètre physiologique chez une personne portant l'équipement optique, le paramètre physiologique indiquant la variation de l'erreur de réfraction courante chez la personne par rapport à l'erreur de réfraction initiale.

5. Procédé selon la revendication 4, dans lequel:
- le capteur physiologique est un capteur de plissement des yeux,
- le paramètre physiologique est une manifestation de plissement des yeux chez la personne portant l'équipement optique, et
- la mesure (S2) de la valeur courante dudit paramètre comprend la détection, à l'aide du capteur de plissement des yeux, d'une manifestation de plissement des yeux chez la personne portant l'équipement optique.

6. Procédé selon la revendication 4, dans lequel :
- le capteur physiologique comporte au moins une électrode adaptée à l'électroencéphalographie,
- chacune de l'au moins une électrode est agencée de manière à être au contact d'une zone frontale ou occipitale de la personne lorsque celle-ci porte l'équipement optique,
- le paramètre physiologique est une activité électrique cérébrale chez la personne portant l'équipement optique, et
- la mesure (S2) de la valeur courante dudit paramètre comprend l'analyse, à l'aide de l'au moins une électrode, de l'activité électrique cérébrale chez la personne portant l'équipement optique.

7. Procédé selon l'une quelconque des revendications 3 à 6, dans lequel :
- la mémoire stocke une indication d'une condition d'observation de référence,
- l'équipement optique comprend en outre au moins un deuxième capteur qui, lorsque l'équipement optique est porté par la personne et que celle-ci observe une scène à travers l'équipement optique, est adapté à obtenir une indication d'une condition d'observation courante,
- le procédé comprenant en outre, l'équipement optique étant porté par la personne et la personne observant la scène à travers l'équipement optique, l'obtention (S3), par le deuxième capteur, de l'indication de la condition d'observation courante, et
- la détection (S7) d'une variation d'une erreur de réfraction courante chez la personne par rapport à l'erreur de réfraction initiale étant basée en outre sur une comparaison entre l'indication de la condition d'observation courante et l'indication de la condition d'observation de référence.

8. Procédé selon la revendication 7, dans lequel au moins un deuxième capteur est un capteur de conditions de luminosité et le paramètre de condition d'observation indique une intensité lumineuse de la scène observée par la personne à travers la lentille réfractive active pendant la mesure, par le capteur physiologique, d'une valeur courante du paramètre physiologique.

9. Procédé selon la revendication 7, dans lequel au moins un deuxième capteur est un capteur de distance et le paramètre de condition d'observation indique une distance entre la lentille réfractive active et un objet de la scène observée par la personne à travers la lentille réfractive active pendant la mesure, par le capteur physiologique, d'une valeur courante du paramètre physiologique.

10. Procédé selon l'une quelconque des revendications 3 à 9, dans lequel la mesure (S2) de la valeur courante dudit paramètre et la détection (S7) de la variation de l'amétropie courante chez la personne par rapport à l'amétropie initiale sont répétées dans le temps.

11. Procédé de réglage d'une fonction dioptrique d'une lentille réfractive active, comprenant :
a) l'obtention (S1) d'un équipement optique transparent comprenant une lentille réfractive active, une fonction dioptrique de la lentille réfractive active étant adaptée à corriger une erreur de réfraction initiale chez une personne, l'équipement optique comprenant en outre un circuit de traitement comprenant un processeur relié fonctionnellement à une mémoire et à une interface de communication avec la lentille réfractive active,
b) à l'aide de la mémoire, l'obtention (S6) d'une valeur courante d'un paramètre, la valeur courante indiquant une variation d'une erreur de réfraction courante chez la personne par rapport à l'erreur de réfraction initiale,
c) à l'aide du circuit de traitement, la détection (S7) d'une variation d'une erreur de réfraction courante chez la personne par rapport à l'erreur de réfraction initiale sur la base de la valeur courante obtenue du paramètre, et
d) à l'aide du circuit de traitement, le réglage (S8) de la fonction dioptrique de la lentille réfractive active si la variation détectée dépasse un seuil prédéterminé, et le maintien de la fonction dioptrique de la lentille réfractive active si la variation détectée est inférieure ou égale au seuil prédéterminé.

12. Equipement optique comprenant une lentille optique et un circuit de traitement, le circuit de traitement comprenant un processeur (PROC) relié à une mémoire (MEM), le circuit de traitement étant configuré pour mettre en œuvre les étapes b) à d) du procédé selon l'une quelconque des revendications 1 à 11,
l'équipement optique étant transparent et adapté à être porté par une personne.
